(19) Europäisches Patentamt
European Patent Office
Office européen des brevets

(11) EP 2 994 094 B1

(12) # EUROPEAN PATENT SPECIFICATION

(45) Date of publication and mention of the grant of the patent:
**17.06.2020 Bulletin 2020/25**

(21) Application number: **14727349.4**

(22) Date of filing: **06.05.2014**

(51) Int Cl.:
***A61K 8/34*** *(2006.01)* ***A61K 8/41*** *(2006.01)*
***A61K 8/44*** *(2006.01)* ***A61Q 5/02*** *(2006.01)*
***A61Q 5/12*** *(2006.01)* ***A61K 8/49*** *(2006.01)*

(86) International application number:
**PCT/US2014/036912**

(87) International publication number:
**WO 2014/182660 (13.11.2014 Gazette 2014/46)**

(54) **HAIR CARE CONDITIONING COMPOSITION COMPRISING HISTIDINE**

HAARPFLEGEZUSAMMENSETZUNG MIT HISTIDIN

COMPOSITION DE CONDITIONNEMENT POUR SOINS CAPILLAIRES COMPRENANT DE L'HISTIDINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.05.2013 US 201361821455 P**

(43) Date of publication of application:
**16.03.2016 Bulletin 2016/11**

(73) Proprietor: **The Procter & Gamble Company**
**Cincinnati, OH 45202 (US)**

(72) Inventors:
- **MARSH, Jennifer, Mary**
  **Cincinnati, OH 45202 (US)**
- **IWATA, Toshiyuki**
  **Singapore 138547 (SG)**
- **LIM, Phan, Shean**
  **Singapore 138547 (SG)**

(74) Representative: **P&G Patent Germany**
**Procter & Gamble Service GmbH**
**Sulzbacher Straße 40**
**65824 Schwalbach am Taunus (DE)**

(56) References cited:
**WO-A1-00/51555** **WO-A1-93/11737**
**FR-A1- 2 853 531** **US-A- 5 158 684**
**US-A- 5 635 167**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 994 094 B1

## Description

### FIELD OF THE INVENTION

The present invention relates to a hair care conditioning composition that removes minerals from the hair during use.

### BACKGROUND OF THE INVENTION

Many water sources that are used by consumers for personal care contain elevated levels of calcium and magnesium salts, as well as undesirable levels of redox metals (e.g., copper and/or iron) salts. As such, using chelants to sequester trace redox metals often proves to be ineffective because most chelants also competitively bind calcium and/or magnesium.

It has been found that even trace quantities of these minerals can deposit on the hair surface and in between the cuticle layers of hair. This deposition of minerals on hair is especially problematic because transition metal ions, such as copper and iron, can facilitate reduction-oxidation (redox) reactions during hair coloring treatments and during UV exposure. These reactions generate reactive oxygen species (ROS), which in turn can cause damage to the hair. In addition, they can interfere with the oxidative color formation chemistry and lead to reduced color uptake for hair colorant users.

It has also been found that traditional chelating agents such as EDDS, can result in stability problems for conditioners containing cationic surfactants, specifically those with C22 or higher cationic surfactants.

Accordingly, there is a need for hair care compositions that can inhibit minerals depositing on keratinous tissue, as well as facilitate the removal of minerals already deposited thereon. Additionally, there is a need for chelating agents which can facilitate removal of minerals deposited on the hair without interfering with the hair care formulation in which the chelating agent is included.

### SUMMARY OF THE INVENTION

A hair care composition comprising: from 0.025% to 0.25% by weight of the composition of histidine; a gel matrix comprising from 0.1% to 20% of one or more high melting point fatty compounds, by weight of the hair care composition, wherein the one or more high melting point fatty compounds have a melting point of 25°C or higher, and comprise fatty alcohols, wherein the total amount of the one or more high melting point fatty compounds ranges from 0.1% to 20%; from 0.1% to 10% of a cationic surfactant system having 22 carbon atoms, by weight of the hair care composition; and at least 20% of an aqueous carrier, by weight of the hair care composition.

### DETAILED DESCRIPTION OF THE INVENTION

While the specification concludes with claims particularly pointing out and distinctly claiming the invention, it is believed that the present invention will be better understood from the following description.

Herein, "comprising" means that other steps and other ingredients which do not affect the end result can be added. This term encompasses the terms "consisting of' and "consisting essentially of'.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "mixtures" is meant to include a simple combination of materials and any compounds that may result from their combination.

The term "molecular weight" or "M.Wt." as used herein refers to the weight average molecular weight unless otherwise stated.

### Hair Care Composition

The hair composition described herein is a conditioning hair care composition that delivers consumer desired conditioning in addition to inhibiting the deposition of minerals (i.e. from the water used to rinse) on the hair.

It has been found that a chelant having a high Stability Constant for Copper ($K_{CuL}$) in combination with a low Stability Constant for Calcium ($K_{CaL}$) will demonstrate a sufficient level of selective affinity for these redox metals and thus inhibit the deposition of the minerals onto hair. Chelants having this selective affinity may also reduce the quantities of redox metals already deposited. Suitable chelants for high affinity for transition metals such as copper and iron generally have at least one negative charge such as amino carboxylates or amino phosphonates. However, chelants

with a negative charge, for example EDDS, can interfere with the stability of the product and specifically form a precipitate with the cationic surfactants which ultimately can lead to a difficulties maintaining the desired viscosity of the conditioner, making it runny and not acceptable for use. In addition the higher charged anionic materials increase ionic strength of the system which can lead to phase separation. Specifically, the longer chain cationic surfactants (C22) will form a turbid solution when these anionic chelants are added, even at low levels. (See Fig. 1) These longer chain surfactants (C22) will be more sensitive to the addition of anionic chelants than the shorter chain surfactants (C18) due to their higher molecular weight. In addition, the longer chain C22 cationic surfactants are more hydrophobic than C18 cationic surfactants and have lower water solubility. The C22 cationic surfactants are important to deliver a high level of wet hair feel conditioning performance because the structures formed have a higher percentage of lamella structure which drives deposition of silicone and the surfactant/fatty alcohol gel matrix which ultimately leads to improved wet conditioning performance vs. the C18 cationic surfactants. Thus there is a need to identify a chelant that can inhibit the deposition of minerals in hair but not interfere with the stability of the conditioner and its wet conditioning performance and deliver superior conditioning performance

## A. HISTIDINE

It has been found that histidine has the high stability constant for copper and low stability constant for calcium that is desired for efficient inhibition of deposition of minerals and can be formulated up to a level of 0.25% in conditioners made with C22 or higher surfactants to give a stable product with no negative impact on conditioning performance. Histidine can be either zwitterionic or uncharged at the pH of a typical hair conditioning composition (pH 4-6) and thus have limited interaction with the C22 or higher surfactants. This enables the formulation of a stable conditioner with histidine at a level of from 0.025% to 0.25%, from 0.05 to 0.25%, from 0.08 to 0.15, and/or from 0.10 to 0.15. Histidine is included at levels sufficient to deliver adequate copper removal performance without interfering with conditioning performance.

The Stability Constant of a metal chelant interaction is defined as:

$$K_{ML} = \frac{[ML]}{[M][L]}$$

where:

[ML] = concentration of metal ligand complex at equilibrium
[M] = concentration of free metal ion
[L] = concentration of free ligand in a fully deprotonated form
$K_{ML}$ = stability constant for the metal chelant complex.

All concentrations are expressed in mol/dm$^3$. Stability constants are conveniently expressed as logarithms.

Histidine may contain a chiral center and may be present in the D- and L- form. For present compositions either form may be acceptable as may be a mixture of the D- and L-forms.

A person skilled in the art could manufacture histidine using standard techniques. See, for example, Organic Chemistry, Fifth Edition, TW Graham Soloman, John Wiley & Son Inc (1992) p 1092-1136

## B. CATIONIC SURFACTANT SYSTEM

The composition of the present invention comprises a cationic surfactant system. The cationic surfactant system can be one cationic surfactant or a mixture of two or more cationic surfactants. The cationic surfactant system can be selected from: mono-long alkyl quaternized ammonium salt; a combination of mono-long alkyl quaternized ammonium salt and di-long alkyl quaternized ammonium salt; mono-long alkyl amidoamine salt; a combination of mono-long alkyl amidoamine salt and di-long alkyl quaternized ammonium salt, a combination of mono-long alkyl amindoamine salt and mono-long alkyl quaternized ammonium salt.

The cationic surfactant system can be included in the composition at a level by weight of from 0.1% to 10%, from 0.5% to 8%, from 0.8 % to 5%, and from 1.0% to 4%.

### Mono-long alkyl quaternized ammonium salt

The monoalkyl quaternized ammonium salt cationic surfactants useful herein are those having one long alkyl

chain which has 22 carbon atoms and in one embodiment a C22 alkyl group. The remaining groups attached to nitrogen are independently selected from an alkyl group of from 1 to 4 carbon atoms or an alkoxy, polyoxyalkylene, alkylamido, hydroxyalkyl, aryl or alkylaryl group having up to 4 carbon atoms.

Mono-long alkyl quaternized ammonium salts useful herein are those having the formula (I):

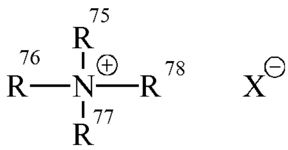

(I)

wherein one of $R^{75}$, $R^{76}$, $R^{77}$ and $R^{78}$ is selected from an alkyl group of 22 carbon atoms or an aromatic, alkoxy, polyoxyalkylene, alkylamido, hydroxyalkyl, aryl or alkylaryl group having up to 30 carbon atoms; the remainder of $R^{75}$, $R^{76}$, $R^{77}$ and $R^{78}$ are independently selected from an alkyl group of from 1 to 4 carbon atoms or an alkoxy, polyoxyalkylene, alkylamido, hydroxyalkyl, aryl or alkylaryl group having up to 4 carbon atoms; and $X^-$ is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of 22 carbons, or higher, can be saturated or unsaturated. One of $R^{75}$, $R^{76}$, $R^{77}$ and $R^{78}$ can be selected from an alkyl group of 22 carbon atoms, the remainder of $R^{75}$, $R^{76}$, $R^{77}$ and $R^{78}$ are independently selected from $CH_3$, $C_2H_5$, $C_2H_4OH$, and mixtures thereof; and X is selected from the group consisting of Cl, Br, $CH_3OSO_3$, $C_2H_5OSO_3$, and mixtures thereof.

Nonlimiting examples of such mono-long alkyl quaternized ammonium salt cationic surfactants include: behenyl trimethyl ammonium salt.

## Mono-long alkyl amidoamine salt

Mono-long alkyl amines are also suitable as cationic surfactants. Primary, secondary, and tertiary fatty amines are useful. Particularly useful are tertiary amido amines having an alkyl group of 22 carbons. Exemplary tertiary amido amines include: behenamidopropyldimethylamine, behenamidopropyldiethylamine, behenamidoethyldiethylamine, behenamidoethyldimethylamin. Useful amines in the present invention are disclosed in U.S. Patent 4,275,055, Nachtigal, et al. These amines can also be used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; in one embodiment ℓ-glutamic acid, lactic acid, and/or citric acid. The amines herein can be partially neutralized with any of the acids at a molar ratio of the amine to the acid of from 1 : 0.3 to 1 : 2, and/or from 1 : 0.4 to 1 : 1.

## Di-long alkyl quaternized ammonium salt

Di-long alkyl quaternized ammonium salt can be combined with a mono-long alkyl quaternized ammonium salt or mono-long alkyl amidoamine salt. It is believed that such combination can provide easy-to rinse feel, compared to single use of a monoalkyl quaternized ammonium salt or mono-long alkyl amidoamine salt. In such combination with a mono-long alkyl quaternized ammonium salt or mono-long alkyl amidoamine salt, the di-long alkyl quaternized ammonium salts are used at a level such that the wt% of the dialkyl quaternized ammonium salt in the cationic surfactant system is in the range of from 10% to 50%, and/or from 30% to 45%.

The di-long alkyl quaternized ammonium salt cationic surfactants useful herein are those having two long alkyl chains having 22 carbon atoms. The remaining groups attached to nitrogen are independently selected from an alkyl group of from 1 to 4 carbon atoms or an alkoxy, polyoxyalkylene, alkylamido, hydroxyalkyl, aryl or alkylaryl group having up to 4 carbon atoms.

Di-long alkyl quaternized ammonium salts useful herein are those having the formula (II):

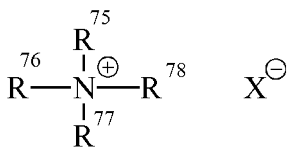

(II)

wherein two of $R^{75}$, $R^{76}$, $R^{77}$ and $R^{78}$ is selected from an alkyl group of from 22 carbon atoms or an aromatic, alkoxy,

polyoxyalkylene, alkylamido, hydroxyalkyl, aryl or alkylaryl group having up to 30 carbon atoms; the remainder of $R^{75}$, $R^{76}$, $R^{77}$ and $R^{78}$ are independently selected from an alkyl group of from 1 to 4 carbon atoms or an alkoxy, polyoxyalkylene, alkylamido, hydroxyalkyl, aryl or alkylaryl group having up to 4 carbon atoms; and $X^-$ is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of 22 carbons, or higher, can be saturated or unsaturated. One of $R^{75}$, $R^{76}$, $R^{77}$ and $R^{78}$ can be selected from an alkyl group of from 22 carbon atoms, the remainder of $R^{75}$, $R^{76}$, $R^{77}$ and $R^{78}$ are independently selected from $CH_3$, $C_2H_5$, $C_2H_4OH$, and mixtures thereof; and X is selected from the group consisting of Cl, Br, $CH_3OSO_3$, $C_2H_5OSO_3$, and mixtures thereof.

Such dialkyl quaternized ammonium salt cationic surfactants include, for example, dialkyl (C22) dimethyl ammonium chloride, ditallow alkyl dimethyl ammonium chloride, dihydrogenated tallow alkyl dimethyl ammonium chloride. Such dialkyl quaternized ammonium salt cationic surfactants also include, for example, asymmetric dialkyl quaternized ammonium salt cationic surfactants.

## C. HIGH MELTING POINT FATTY COMPOUND

The high melting point fatty compound useful herein have a melting point of 25°C or higher, and comprise fatty alcohols. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain carbon atoms may have a melting point of less than 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols are suitable for use in the composition of the present invention. The fatty alcohols useful herein are those having from 14 to 30 carbon atoms, from 16 to 22 carbon atoms. These fatty alcohols are saturated and can be straight or branched chain alcohols. Suitable fatty alcohols include, for example, cetyl alcohol, stearyl alcohol, behenyl alcohol, and mixtures thereof.

High melting point fatty compounds of a single compound of high purity can be used. Single compounds of pure fatty alcohols selected from the group of pure cetyl alcohol, stearyl alcohol, and behenyl alcohol can also be used. By "pure" herein, what is meant is that the compound has a purity of at least 90%, and/or at least 95%. These single compounds of high purity provide good rinsability from the hair when the consumer rinses off the composition.

The high melting point fatty compound can be included in the composition at a level of from 0.1% to 20%, from 1% to 15%, and/or from 1.5% to 8% by weight of the composition, in view of providing improved conditioning benefits such as slippery feel during the application to wet hair, softness and moisturized feel on dry hair.

## D. AQUEOUS CARRIER

The gel matrix of the hair care composition of the present invention includes an aqueous carrier. Accordingly, the formulations of the present invention can be in the form of pourable liquids (under ambient conditions). Such compositions will therefore typically comprise an aqueous carrier, which is present at a level of from 20 wt% to 95 wt%, or from 60 wt% to 85 wt%. The aqueous carrier may comprise water, or a miscible mixture of water and organic solvent, and in one aspect may comprise water with minimal or no significant concentrations of organic solvent, except as otherwise incidentally incorporated into the composition as minor ingredients of other components.

The aqueous carrier useful in the present invention includes water and water solutions of lower alkyl alcohols and polyhydric alcohols. The lower alkyl alcohols useful herein are monohydric alcohols having 1 to 6 carbons, in one aspect, ethanol and isopropanol. The polyhydric alcohols useful herein include propylene glycol, hexylene glycol, glycerin, and propane diol.

According to embodiments of the present invention, the hair care compositions may have a pH in the range from 2 to 10, at 25°C. In one embodiment, the hair care composition has a pH in the range from 2 to 6, which may help to solubilize minerals and redox metals already deposited on the hair. Thus, the hair care composition can also be effective toward washing out the existing minerals and redox metals deposits, which can reduce cuticle distortion and thereby reduce cuticle chipping and damage.

## E. GEL MATRIX

The composition of the present invention comprises a gel matrix. The gel matrix comprises a cationic surfactant,

a high melting point fatty compound, and an aqueous carrier.
The gel matrix is suitable for providing various conditioning benefits such as slippery feel during the application to wet hair and softness and moisturized feel on dry hair. In view of providing the above gel matrix, the cationic surfactant and the high melting point fatty compound are contained at a level such that the weight ratio of the cationic surfactant to the high melting point fatty compound is in the range of, from 1:1 to 1:10, and/or from 1:1 to 1:6.

## F. ADDITIONAL COMPONENTS

1. Silicone Conditioning Agent

According to embodiments of the present invention, the hair care composition includes a silicone conditioning agent which comprises a silicone compound. The silicone compound may comprise volatile silicone, non-volatile silicones, or combinations thereof. In one aspect, non-volatile silicones are employed. If volatile silicones are present, it will typically be incidental to their use as a solvent or carrier for commercially available forms of non-volatile silicone materials ingredients, such as silicone gums and resins. The silicone compounds may comprise a silicone fluid conditioning agent and may also comprise other ingredients, such as a silicone resin to improve silicone fluid deposition efficiency or enhance glossiness of the hair. The concentration of the silicone compound in the conditioner composition typically ranges from 0.01 wt% to 10 wt%, from 0.1 wt% to 8 wt%, from 0.1 wt% to 5 wt%, or even from 0.2 wt% to 3 wt%, for example

Exemplary silicone compounds include (a) a first polysiloxane which is non-volatile, substantially free of amino groups, and has a viscosity of from 100,000 $mm^2s^{-1}$ to 30,000,000 $mm^2s^{-1}$; (b) a second polysiloxane which is non-volatile, substantially free of amino groups, and has a viscosity of from 5 $mm^2s^{-1}$ to 10,000 $mm^2s^{-1}$; (c) an aminosilicone having less than 0.5 wt% nitrogen by weight of the aminosilicone; (d) a silicone copolymer emulsion with an internal phase viscosity of greater than 100x$10^6$ $mm^2s^{-1}$, as measured at 25°C; (e) a silicone polymer containing quaternary groups; or (f) a grafted silicone polyol, wherein the silicone compounds (a) - (f) are disclosed in U.S. Patent Application Publication Nos. 2008/0292574, 2007/0041929, 2008/0292575, and 2007/0286837, each of which is incorporated by reference herein in its entirety.

a. First Polysiloxane

The hair care composition of the present invention may comprise a first polysiloxane. The first polysiloxane is non-volatile, and substantially free of amino groups. In the present invention, the first polysiloxanes being "substantially free of amino groups" means that the first polysiloxane contains 0 wt% of amino groups. The first polysiloxane has a viscosity of from 100,000 $mm^2s^{-1}$ to 30,000,000 $mm^2s^{-1}$ at 25°C. For example, the viscosity may range from 300,000 $mm^2s^{-1}$ to 25,000,000 $mm^2s^{-1}$, or from 10,000,000 $mm^2s^{-1}$ to 20,000,000 $mm^2s^{-1}$. The first polysiloxane has a molecular weight from 100,000 to 1,000,000. For example, the molecular weight may range from 130,000 to 800,000, or from 230,000 to 600,000. According to one aspect, the first polysiloxane may be nonionic.

Exemplary first non-volatile polysiloxanes useful herein include those in accordance with the following the general formula (I):

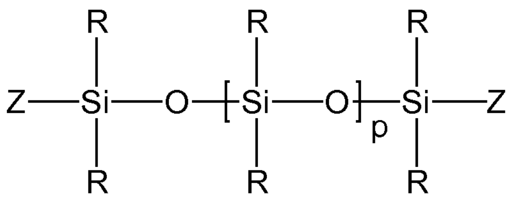

(I)

wherein R is alkyl or aryl, and p is an integer from 1,300 to 15,000, such as from 1,700 to 11,000, or from 3,000 to 8,000. Z represents groups which block the ends of the silicone chains. The alkyl or aryl groups substituted on the siloxane chain (R) or at the ends of the siloxane chains Z can have any structure as long as the resulting silicone remains fluid at room temperature, is dispersible, is neither irritating, toxic nor otherwise harmful when applied to the hair, is compatible with the other components of the composition, is chemically stable under normal use and storage conditions, and is capable of being deposited on and conditions the hair. According to an embodiment, suitable Z groups include hydroxy, methyl, methoxy, ethoxy, propoxy, and aryloxy. The two R groups on each silicon atom may represent the same group or different groups. According to one embodiment, the two R groups may represent the same group. Suitable R groups include methyl, ethyl, propyl, phenyl, methylphenyl and phenylmethyl. Exemplary silicone compounds include polydimethylsiloxane, polydiethylsiloxane, and polymethylphenylsiloxane. According to one embodiment, polydimethylsiloxane is the first polysiloxane. Commercially available silicone compounds useful herein include, for example, those

available from the General Electric Company in their TSF451 series, and those available from Dow Corning in their Dow Corning SH200 series.

The silicone compounds that can be used herein also include a silicone gum. The term "silicone gum", as used herein, means a polyorganosiloxane material having a viscosity at 25°C of greater than or equal to 1,000,000 $mm^2s^{-1}$. It is recognized that the silicone gums described herein can also have some overlap with the above-disclosed silicone compounds. This overlap is not intended as a limitation on any of these materials. The "silicone gums" will typically have a mass molecular weight in excess of 165,000, generally between 165,000 and 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. Commercially available silicone gums useful herein include, for example, TSE200A available from the General Electric Company.

b. Second Polysiloxane

The hair care composition of the present invention may comprise a second polysiloxane. The second polysiloxane is non-volatile, and substantially free of amino groups. In the present invention, the second polysiloxane being "substantially free of amino groups" means that the second polysiloxane contains 0 wt% of amino groups. The second polysiloxane has a viscosity of from 5 $mm^2s^{-1}$ to 10,000 $mm^2s^{-1}$ at 25°C, such as from 5 $mm^2s^{-1}$ to 5,000 $mm^2s^{-1}$, from 10 $mm^2s^{-1}$ to 1,000 $mm^2s^{-1}$, or from 20 $mm^2s^{-1}$ to 350 $mm^2s^{-1}$. The second polysiloxane has a molecular weight of from 400 to 65,000. For example, the molecular weight of the second polysiloxane may range from 800 to 50,000, from 400 to 30,000, or from 400 to 15,000. According to one aspect, the second polysiloxane may be nonionic. According to another aspect, the second polysiloxane may be a linear silicone.

Exemplary second non-volatile polysiloxanes useful herein include polyalkyl or polyaryl siloxanes in accordance with the following the general formula (II):

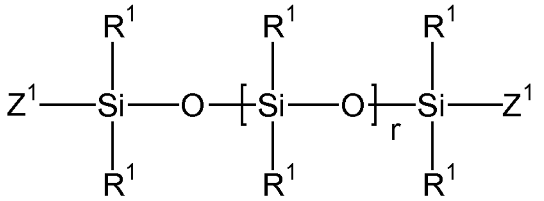

(II)

wherein $R^1$ is alkyl or aryl, and r is an integer from 7 to 850, such as from 7 to 665, from 7 to 400, or from 7 to 200. $Z^1$ represents groups which block the ends of the silicone chains. The alkyl or aryl groups substituted on the siloxane chain ($R^1$) or at the ends of the siloxane chains $Z^1$ can have any structure as long as the resulting silicone remains fluid at room temperature, is dispersible, is neither irritating, toxic nor otherwise harmful when applied to the hair, is compatible with the other components of the composition, is chemically stable under normal use and storage conditions, and is capable of being deposited on and conditions the hair. According to an embodiment, suitable $Z^1$ groups include hydroxy, methyl, methoxy, ethoxy, propoxy, and aryloxy. The two $R^1$ groups on each silicon atom may represent the same group or different groups. According to one embodiment, the two $R^1$ groups may represent the same group. Suitable $R^1$ groups include methyl, ethyl, propyl, phenyl, methylphenyl and phenylmethyl. Exemplary silicone compounds include polydimethylsiloxane, polydiethylsiloxane, and polymethylphenylsiloxane. According to one embodiment, polydimethylsiloxane is the second polysiloxane. Commercially available silicone compounds useful herein include, for example, those available from the General Electric Company in their TSF451 series, and those available from Dow Corning in their Dow Corning SH200 series.

c. Amino silicone

The hair care composition of the present invention may comprise an amino silicone having less than 0.5 wt% nitrogen by weight of the aminosilicone, such as less than 0.2 wt%, or less than 0.1 wt%, in view of friction reduction benefit. It has been surprisingly found that higher levels of nitrogen (amine functional groups) in the amino silicone tend to result in less friction reduction, and consequently less conditioning benefit from the aminosilicone. The aminosilicone useful herein may have at least one silicone block with greater than 200 siloxane units, in view of friction reduction benefit. The amino silicones useful herein include, for example, quaternized aminosilicone and non-quaternized aminosilicone.

In one embodiment, the aminosilicones useful herein are water-insoluble. In the present invention, "water-insoluble aminosilicone" means that the aminosilicone has a solubility of 10g or less per 100g water at 25°C, in another embodiment 5g or less per 100g water at 25°C, and in another embodiment 1g or less per 100g water at 25°C. In the

present invention, "water-insoluble aminosilicone" means that the aminosilicone is substantially free of copolyol groups. If copolyol groups are present, they are present at a level of less than 10 wt%, less than 1 wt%, or less than 0.1 wt% by weight of the aminosilicone.

According to one embodiment, aminosilicone useful herein are those which conform to the general formula (III):

$$(R^2)_aG_{3-a}\text{-Si(-O-SiG}_2)_n\text{(-O-SiG}_b(R^2)_{2-b})_m\text{-O-SiG}_{3-a}(R^2)_a \quad \text{(IIII)}$$

wherein G is hydrogen, phenyl, hydroxy, or $C_1$-$C_8$ alkyl, such as methyl; a is an integer having a value from 1 to 3, such as 1; b is an integer having a value from 0 to 2, such as 1; n is a number from 1 to 2,000, such as from 100 to 1,800, from 300 to 800, or from 500 to 600; m is an integer having a value from 0 to 1,999, such as from 0 to 10, or 0; $R^2$ is a monovalent radical conforming to the general formula $C_qH_{2q}L$, wherein q is an integer having a value from 2 to 8 and L is selected from the following groups: $-N(R^3{}_2)CH_2-CH_2-N(R^3{}_2)_2$; $-N(R^3)_2$; $-N^+(R^3)_3A^-$; $-N(R^3)CH_2-CH_2-N^+R^3H_2A^-$; wherein $R^3$ is hydrogen, phenyl, benzyl, or a saturated hydrocarbon radical, such as an alkyl radical from $C_1$ to $C_{20}$; $A^-$ is a halide ion. According to an embodiment, L is $-N(CH_3)_2$ or $-NH_2$. According to another embodiment, L is $-NH_2$.

The aminosilicone of the above formula is used at levels by weight of the composition of from 0.1 wt% to 5 wt%, alternatively from 0.2 wt% to 2 wt%, alternatively from 0.2 wt% to 1.0 wt%, and alternatively from 0.3 wt% to 0.8 wt%.

According to one embodiment, the aminosilicone may include those compounds corresponding to formula (III) wherein m=0; a=1; q=3; G=methyl; n is from 1400 to 1700, such as 1600; and L is $-N(CH_3)_2$ or $-NH_2$, such as $-NH_2$. According to another embodiment, the aminosilicone may include those compounds corresponding to formula (III) wherein m=0; a=1; q=3; G=methyl; n is from 400 to 800, such as from 500 to around 600; and L is L is $-N(CH_3)_2$ or $-NH_2$, such as $-NH_2$. Accordingly, the aforementioned aminosilicones can be called terminal aminosilicones, as one or both ends of the silicone chain are terminated by nitrogen containing group. Such terminal aminosilicones may provide improved friction reduction compared to graft aminosilicones.

Another example of an aminosilicone useful herein includes, for example, quaternized aminosilicone having a tradename KF8020 available from Shinetsu.

The above aminosilicones, when incorporated into the hair care composition, can be mixed with solvent having a lower viscosity. Such solvents include, for example, polar or non-polar, volatile or non-volatile oils. Such oils include, for example, silicone oils, hydrocarbons, and esters. Among such a variety of solvents, exemplary solvents include those selected from the group consisting of non-polar, volatile hydrocarbons, volatile cyclic silicones, non-volatile linear silicones, and mixtures thereof. The non-volatile linear silicones useful herein are those having a viscosity of from 1 $mm^2s^{-1}$ to 20,000 $mm^2s^{-1}$, such as from 20 $mm^2s^{-1}$ to 10,000 $mm^2s^{-1}$, at 25°C. According to one embodiment, the solvents are non-polar, volatile hydrocarbons, especially non-polar, volatile isoparaffins, in view of reducing the viscosity of the aminosilicones and providing improved hair conditioning benefits such as reduced friction on dry hair. Such mixtures may have a viscosity of from 1,000 mPa.s to 100,000 mPa.s, and alternatively from 5,000 mPa.s to 50,000 mPa.s.

d. Silicone Copolymer Emulsion

The hair care composition of the present invention may comprise a silicone copolymer emulsion with an internal phase viscosity of greater than 100 x $10^6$ $mm^2s^{-1}$. The silicone copolymer emulsion may be present in an amount of from 0.1 wt% to 15 wt%, alternatively from 0.3 wt% to 10 wt%, and alternatively 0.5 wt% to 5 wt%, by weight of the composition, in view of providing clean feel.

According to one embodiment, the silicone copolymer emulsion has a viscosity at 25°C of greater than 100 x $10^6$ $mm^2s^{-1}$, alternatively greater than 120 x $10^6$ $mm^2s^{-1}$, and alternatively greater than 150 x $10^6$ $mm^2s^{-1}$ According to another embodiment, the silicone copolymer emulsion has a viscosity at 25°C of less than 1000 x $10^6$ $mm^2s^{-1}$, alternatively less than 500 x $10^6$ $mm^2s^{-1}$, and alternatively less than 300 x $10^6$ $mm^2s^{-1}$. To measure the internal phase viscosity of the silicone copolymer emulsion, one may first break the polymer from the emulsion. By way of example, the following procedure can be used to break the polymer from the emulsion: 1) add 10 grams of an emulsion sample to 15 milliliters of isopropyl alcohol; 2) mix well with a spatula; 3) decant the isopropyl alcohol; 4) add 10 milliliters of acetone and knead polymer with spatula; 5) decant the acetone; 6) place polymer in an aluminum container and flatten/dry with a paper towel; and 7) dry for two hours in an 80°C. The polymer can then be tested using any known rheometer, such as, for example, a CarriMed, Haake, or Monsanto rheometer, which operates in the dynamic shear mode. The internal phase viscosity values can be obtained by recording the dynamic viscosity (n') at a 9.900*$10^{-3}$ Hz frequency point. According to one embodiment, the average particle size of the emulsions is less than 1 micron, such as less than 0.7 micron.

The silicone copolymer emulsions of the present invention may comprise a silicone copolymer, at least one surfactant, and water.

The silicone copolymer results from the addition reaction of the following two materials in the presence of a metal containing catalyst:

(i) a polysiloxane with reactive groups on both termini, represented by a general formula (IV):

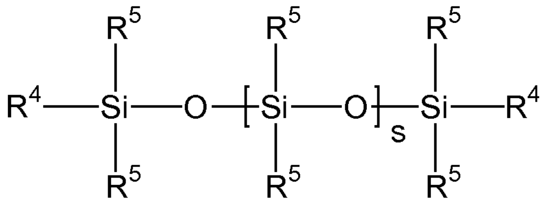

(IV)

wherein:

$R^4$ is a group capable of reacting by chain addition reaction such as, for example, a hydrogen atom, an aliphatic group with ethylenic unsaturation (i.e., vinyl, allyl, or hexenyl), a hydroxyl group, an alkoxyl group (i.e., methoxy, ethoxy, or propoxy), an acetoxyl group, or an amino or alkylamino group;
$R^5$ is alkyl, cycloalkyl, aryl, or alkylaryl and may include additional functional groups such as ethers, hydroxyls, amines, carboxyls, thiols esters, and sulfonates; in an embodiment, $R^5$ is methyl. Optionally, a small mole percentage of the groups may be reactive groups as described above for $R^5$, to produce a polymer which is substantially linear but with a small amount of branching. In this case, the level of $R^5$ groups equivalent to $R^4$ groups may be less than 10% on a mole percentage basis, such as less than 2%;
s is an integer having a value such that the polysiloxane of formula (IV) has a viscosity of from 1 $mm^2s^{-1}$ to 1 x $10^6$ $mm^2s^{-1}$;
and,

(ii) at least one silicone compound or non-silicone compound comprising at least one or at most two groups capable of reacting with the $R^4$ groups of the polysiloxane in formula (IV). According to one embodiment, the reactive group is an aliphatic group with ethylenic unsaturation.

The metal containing catalysts used in the above described reactions are often specific to the particular reaction. Such catalysts are known in the art. Generally, they are materials containing metals such as platinum, rhodium, tin, titanium, copper, lead, etc.

The mixture used to form the emulsion also may contain at least one surfactant. This can include non-ionic surfactants, cationic surfactants, anionic surfactants, alkylpolysaccharides, amphoteric surfactants, and the like. The above surfactants can be used individually or in combination.

An exemplary method of making the silicone copolymer emulsions described herein comprises the steps of 1) mixing materials (a) described above with material (b) described above, followed by mixing in an appropriate metal containing catalyst, such that material (b) is capable of reacting with material (a) in the presence of the metal containing catalyst; 2) further mixing in at least one surfactant and water; and 3) emulsifying the mixture. Methods of making such silicone copolymer emulsions are disclosed in U.S. Pat. No. 6,013,682; PCT Application No. WO 01/58986 A1; and European Patent Application No. EP0874017 A2.

A commercially available example of a silicone copolymer emulsion is an emulsion of 60-70 wt% of divinyldimethicone/dimethicone copolymer having an internal phase viscosity of minimum 120 x $10^6$ $mm^2s^{-1}$, available from Dow Corning with a tradename HMW2220.

e. Silicone polymer containing quaternary groups

The hair care composition of the present invention may comprise a silicone polymer containing quaternary groups (i.e., a quaternized silicone polymer). The quaternized silicone polymer provides improved conditioning benefits such as smooth feel, reduced friction, prevention of hair damage. Especially, the quaternary group can have good affinity with damaged/colorant hairs. The quaternized silicone polymer is present in an amount of from 0.1 wt% to 15 wt%, based on the total weight of the hair conditioning composition. For example, according to an embodiment, the quaternized silicone polymer may be present in an amount from 0.2 wt% to 10 wt%, alternatively from 0.3 wt% to 5 wt%, and alternatively from 0.5 wt% to 4 wt%, by weight of the composition.

The quaternized silicone polymer of the present invention is comprised of at least one silicone block and at least one non-silicone block containing quaternary nitrogen groups, wherein the number of the non-silicone blocks is one greater than the number of the silicone blocks. The silicone polymers correspond to the general structure (V):

$$A^1\text{-}B\text{-}(A^2\text{-}B)_m\text{-}A^1 \quad (V)$$

wherein, B is a silicone block having greater than 200 siloxane units; $A^1$ is an end group which may contain quaternary groups; $A^2$ is a non-silicone blocks containing quaternary nitrogen groups; and m is an integer 0 or greater, with the proviso that if m=0 then the $A^1$ group contains quaternary groups.

Structures corresponding to the general formula, for example, are disclosed in U.S. Pat. No. 4,833,225, in U.S. Patent Application Publication No. 2004/0138400, in U.S. Patent Application Publication No. 2004/0048996, and in U.S. Patent Application Publication No. 2008/0292575.

In one embodiment, the silicone polymers can be represented by the following structure (VI)

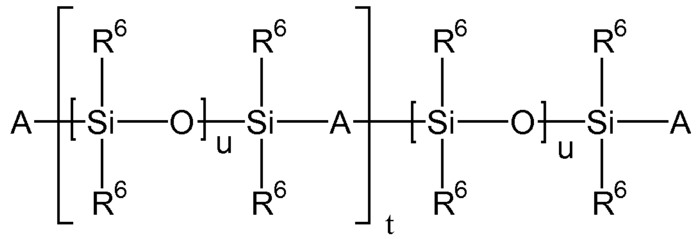

(VI)

wherein, A is a group which contains at least one quaternary nitrogen group, and which is linked to the silicon atoms of the silicone block by a silicon-carbon bond, each A independently can be the same or different; $R^6$ is an alkyl group of from 1 to 22 carbon atoms or an aryl group; each $R^6$ independently can be the same or different; t is an integer having a value of from 0 or greater, for example t can be less than 20, or less than 10; and u is an integer greater than 200, such as greater than 250, or greater than 300, and u may be less than 700, or less than 500. According to an embodiment, $R^6$ is methyl.

f. Grafted Silicone Copolyol

The hair care composition of the present invention may comprise a grafted silicone copolyol in combination with the quaternized silicone polymer. It is believed that this grafted silicone copolyol can improve the spreadability of the quaternized silicone polymer by reducing the viscosity of the quaternized silicone polymer, and also can stabilize the quaternized silicone polymer in aqueous conditioner matrix. It is also believed that, by such improved spreadability, the hair care compositions of the present invention can provide better dry conditioning benefits such as friction reduction and/or prevention of damage with reduced tacky feel. It has been surprisingly found that the combination of the quaternized silicone polymer, grafted silicone copolyol, and cationic surfactant system comprising di-alkyl quaternized ammonium salt cationic surfactants provides improved friction reduction benefit, compared to a similar combination. Such similar combinations are, for example, a combination in which the grafted silicone copolyol is replaced with end-capped silicone copolyol, and another combination in which the cationic surfactant system is substantially free of di-alkyl quaternized ammonium salt cationic surfactants.

The grafted silicone copolyol is contained in the composition at a level such that the weight % of the grafted silicone copolyol to its mixture with quaternized silicone copolymer is in the range of from 1 wt% to 50 wt%, alternatively from 5 wt% to 40 wt%, and alternatively from 10 wt% to 30 wt%.

The grafted silicone copolyols useful herein are those having a silicone backbone such as dimethicone backbone and polyoxyalkylene substitutions such as polyethylene oxide and/or polypropylene oxide substitutions. The grafted silicone copolyols useful herein have a hydrophilic-lipophilic balance (HLB) value of from 5 to 17, such as from 8 to 17, or from 8 to 12. The grafted silicone copolyols having the same INCI name have a variety of the weight ratio, depending on the molecular weight of the silicone portion and the number of the polyethylene oxide and/or polypropylene oxide substitutions.

According to an embodiment, exemplary commercially available grafted dimethicone copolyols include, for example: those having a tradename Silsoft 430 having an HLB value of from 9 to 12 (INCI name "PEG/PPG-20/23 dimethicone") available from GE; those having a tradename Silsoft 475 having an HLB value of from 13 to 17 (INCI name "PEG-23/PPG-6 dimethicone"); those having a tradename Silsoft 880 having an HLB value of from 13 to 17 (INCI name "PEG-12 dimethicone"); those having a tradename Silsoft 440 having an HLB value of from 9 to 12 (INCI name "PEG-20/PPG-23 dimethicone"); those having a tradename DC5330 (INCI name "PEG-15/PPG-15 dimethicone") available from Dow Corning.

The above quaternized silicone polymer and the grafted silicone copolyol may be mixed and emulsified by a emulsifying surfactant, prior to incorporating them into a gel matrix formed by cationic surfactants and high melting point fatty compounds, as discussed below. It is believed that, this pre-mixture can improve behavior of the quaternized silicone polymer and the grafted silicone copolyol, for example, increase the stability and reduce the viscosity to form more

homogenized formulation together with the other components. Such emulsifying surfactant can be used at a level of 0.001 wt% to 1.5 wt%, alternatively from 0.005% to 1.0%, and alternatively from 0.01 wt% to 0.5 wt%, based on the total weight of the hair conditioning composition. Such surfactants may be nonionic, and have an HLB value of from 2 to 15, such as from 3 to 14, or from 3 to 10. Commercially available examples of emulsifying surfactant include nonionic surfactants having an INCI name C12-C14 Pareth-3 and having an HLB value of 8 supplied from NIKKO Chemicals Co., Ltd. with tradename NIKKOL BT-3.

According to one embodiment, the hair care composition comprises a combination of two or more silicone conditioning agents, along with an EDDS sequestering agent and a gel matrix.

In one embodiment, the hair care composition comprises a polyalkylsiloxane mixture comprising (i) a first polyalkylsiloxane which is non-volatile, substantially free of amino groups, and has a viscosity of from 100,000 $mm^2s^{-1}$ to 30,000,000 $mm^2s^{-1}$, and (ii) a second polyalkylsiloxane which is non-volatile, substantially free of amino groups, and has a viscosity of from 5 $mm^2s^{-1}$ to 10,000 $mm^2s^{-1}$; an aminosilicone having less than 0.5 wt% nitrogen by weight of the aminosilicone; and a silicone copolymer emulsion with an internal phase viscosity of greater than 100 x $10^6$ $mm^2s^{-1}$, as measured at 25°C. For example, in another embodiment, the hair care composition comprises from 0.5 wt% to 10 wt% of a polyalkylsiloxane mixture comprising (i) a first polyalkylsiloxane which is non-volatile, substantially free of amino groups, and has a viscosity of from 100,000 $mm^2s^{-1}$ to 30,000,000 $mm^2s^{-1}$, and (ii) a second polyalkylsiloxane which is non-volatile, substantially free of amino groups, and has a viscosity of from 5 $mm^2s^{-1}$ to 10,000 $mm^2s^{-1}$; from 0.1 wt% to 5 wt% of an aminosilicone having less than 0.5 wt% nitrogen by weight of the aminosilicone; and from 0.1 wt% to 5 wt% of a silicone copolymer emulsion with an internal phase viscosity of greater than 100 x $10^6$ $mm^2s^{-1}$, as measured at 25°C.

In another embodiment, the hair care composition comprises a silicone polymer containing quaternary groups wherein said silicone polymer comprises silicone blocks with greater than 200 siloxane units; and a grafted silicone copolyol. For example, in another embodiment, the hair care composition comprises from 0.1 wt% to 15 wt% of a silicone polymer containing quaternary groups wherein said silicone polymer comprises silicone blocks with greater than 200 siloxane units; and a grafted silicone copolyol at a level such that the weight % of the grafted silicone copolyol in its mixture with the quaternized silicone polymer is in the range of from 1 wt% to 50 wt%.

In yet another embodiment, the hair care composition comprises an amino silicone having a viscosity of from 1,000 centistokes to 1,000,000 centistokes, and less than 0.5% nitrogen by weight of the aminosilicone; and (2) a silicone copolymer emulsion with an internal phase viscosity of greater than 120 x $10^6$ centistokes, as measured at 25°C.

2. Other Conditioning Agents

Also suitable for use in the hair care compositions herein are the conditioning agents described by the Procter & Gamble Company in U.S. Pat. Nos. 5,674,478, and 5,750,122. Also suitable for use herein are those conditioning agents described in U.S. Pat. Nos. 4,529,586, 4,507,280, 4,663,158, 4,197,865, 4,217, 914, 4,381,919, and 4,422, 853.

a. Organic Conditioning Oils

The hair care compositions of the present invention may also further comprise an organic conditioning oil. According to embodiments of the present invention, the hair care composition may comprise from 0.05 wt% to 3 wt%, from 0.08 wt% to 1.5 wt%, or even from 0.1 wt% to 1 wt%, of at least one organic conditioning oil as the conditioning agent, in combination with other conditioning agents, such as the silicones (described herein). Suitable conditioning oils include hydrocarbon oils, polyolefins, and fatty esters. Suitable hydrocarbon oils include, but are not limited to, hydrocarbon oils having at least 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils are typically from C12 to C19. Branched chain hydrocarbon oils, including hydrocarbon polymers, typically will contain more than 19 carbon atoms. Suitable polyolefins include liquid polyolefins, liquid poly-$\alpha$-olefins, or even hydrogenated liquid poly-$\alpha$-olefins. Polyolefins for use herein may be prepared by polymerization of C4 to C14 or even C6 to C12. Suitable fatty esters include, but are not limited to, fatty esters having at least 10 carbon atoms. These fatty esters include esters with hydrocarbyl chains derived from fatty acids or alcohols (e.g. mono-esters, polyhydric alcohol esters, and di- and tri-carboxylic acid esters). The hydrocarbyl radicals of the fatty esters hereof may include or have covalently bonded thereto other compatible functionalities, such as amides and alkoxy moieties (e.g., ethoxy or ether linkages, etc.).

**3.** Nonionic Polymers

The hair care composition of the present invention may also further comprise a nonionic polymer. According to an embodiment, the conditioning agent for use in the hair care composition of the present invention may include a

polyalkylene glycol polymer. For example, polyalkylene glycols having a molecular weight of more than 1000 are useful herein. Useful are those having the following general formula (VIII):

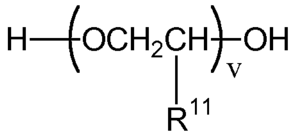

(VIII)

wherein $R^{11}$ is selected from the group consisting of H, methyl, and mixtures thereof; and v is the number of ethoxy units. The polyalkylene glycols, such as polyethylene glycols, can be included in the hair care compositions of the present invention at a level of from 0.001 wt% to 10 wt%. In an embodiment, the polyethylene glycol is present in an amount up to 5 wt% based on the weight of the composition. Polyethylene glycol polymers useful herein are PEG-2M (also known as Polyox WSR® N-10, which is available from Union Carbide and as PEG-2,000); PEG-5M (also known as Polyox WSR® N-35 and Polyox WSR® N-80, available from Union Carbide and as PEG-5,000 and Polyethylene Glycol 300,000); PEG-7M (also known as Polyox WSR® N-750 available from Union Carbide); PEG-9M (also known as Polyox WSR® N-3333 available from Union Carbide); and PEG-14 M (also known as Polyox WSR® N-3000 available from Union Carbide).

4. Suspending Agent

The hair care compositions of the present invention may further comprise a suspending agent at concentrations effective for suspending water-insoluble material in dispersed form in the compositions or for modifying the viscosity of the composition. Such concentrations range from 0.1 wt% to 10 wt%, or even from 0.3 wt% to 5.0 wt%.

Suspending agents useful herein include anionic polymers and nonionic polymers. Useful herein are vinyl polymers such as cross linked acrylic acid polymers with the CTFA name Carbomer, cellulose derivatives and modified cellulose polymers such as methyl cellulose, ethyl cellulose, hydroxyethyl cellulose, hydroxypropyl methyl cellulose, nitro cellulose, sodium cellulose sulfate, sodium carboxymethyl cellulose, crystalline cellulose, cellulose powder, polyvinylpyrrolidone, polyvinyl alcohol, guar gum, hydroxypropyl guar gum, xanthan gum, arabia gum, tragacanth, galactan, carob gum, guar gum, karaya gum, carrageenan, pectin, agar, quince seed (Cydonia oblonga Mill), starch (rice, corn, potato, wheat), algae colloids (algae extract), microbiological polymers such as dextran, succinoglucan, pulleran, starch-based polymers such as carboxymethyl starch, methylhydroxypropyl starch, alginic acid-based polymers such as sodium alginate, alginic acid propylene glycol esters, acrylate polymers such as sodium polyacrylate, polyethylacrylate, polyacrylamide, polyethyleneimine, and inorganic water soluble material such as bentonite, aluminum magnesium silicate, laponite, hectonite, and anhydrous silicic acid.

Commercially available viscosity modifiers highly useful herein include Carbomers with trade names Carbopol® 934, Carbopol® 940, Carbopol® 950, Carbopol® 980, and Carbopol® 981, all available from B. F. Goodrich Company, acrylates/steareth-20 methacrylate copolymer with trade name ACRYSOL™ 22 available from Rohm and Hass, nonoxynyl hydroxyethylcellulose with trade name Amercell™ POLYMER HM-1500 available from Amerchol, methylcellulose with trade name BENECEL®, hydroxyethyl cellulose with trade name NATROSOL®, hydroxypropyl cellulose with trade name KLUCEL®, cetyl hydroxyethyl cellulose with trade name POLYSURF® 67, all supplied by Hercules, ethylene oxide and/or propylene oxide based polymers with trade names CARBOWAX® PEGs, POLYOX WASRs, and UCON® FLUIDS, all supplied by Amerchol.

Other optional suspending agents include crystalline suspending agents which can be categorized as acyl derivatives, long chain amine oxides, and mixtures thereof. These suspending agents are described in U.S. Pat. No. 4,741,855.

These suspending agents include ethylene glycol esters of fatty acids in one aspect having from 16 to 22 carbon atoms. In one aspect, useful suspending agents include ethylene glycol stearates, both mono and distearate, but in one aspect, the distearate containing less than 7% of the mono stearate. Other suitable suspending agents include alkanol amides of fatty acids, having from 16 to 22 carbon atoms, or even 16 to 18 carbon atoms, examples of which include stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc.); long chain esters of long chain alkanol amides (e.g., stearamide diethanolamide distearate, stearamide monoethanolamide stearate); and glyceryl esters (e.g., glyceryl distearate, trihydroxystearin, tribehenin) a commercial example of which is Thixin® R available from Rheox, Inc. Long chain acyl derivatives, ethylene glycol esters of long chain carboxylic acids, long chain amine oxides, and alkanol amides of long chain carboxylic acids in addition to the materials listed above may be used as suspending agents.

Other long chain acyl derivatives suitable for use as suspending agents include N,N-dihydrocarbyl amido

benzoic acid and soluble salts thereof (e.g., Na, K), particularly N,N-di(hydrogenated) C16, C18 and tallow amido benzoic acid species of this family, which are commercially available from Stepan Company (Northfield, Ill., USA).

Examples of suitable long chain amine oxides for use as suspending agents include alkyl dimethyl amine oxides, e.g., stearyl dimethyl amine oxide.

Other suitable suspending agents include primary amines having a fatty alkyl moiety having at least 16 carbon atoms, examples of which include palmitamine or stearamine, and secondary amines having two fatty alkyl moieties each having at least 12 carbon atoms, examples of which include dipalmitoylamine or di(hydrogenated tallow)amine. Still other suitable suspending agents include di(hydrogenated tallow)phthalic acid amide, and crosslinked maleic anhydride-methyl vinyl ether copolymer.

5. Benefit Agents

In an embodiment, the hair care composition further comprises one or more additional benefit agents. The benefit agents comprise a material selected from the group consisting of anti-dandruff agents, vitamins, lipid soluble vitamins, chelants, perfumes, brighteners, enzymes, sensates, attractants, anti-bacterial agents, dyes, pigments, bleaches, and mixtures thereof.

In one aspect said benefit agent may comprise an anti-dandruff agent. Such anti-dandruff particulate should be physically and chemically compatible with the components of the composition, and should not otherwise unduly impair product stability, aesthetics or performance.

According to an embodiment, the hair care composition comprises an anti-dandruff active, which may be an anti-dandruff active particulate. In an embodiment, the anti-dandruff active is selected from the group consisting of: pyridinethione salts; azoles, such as ketoconazole, econazole, and elubiol; selenium sulphide; particulate sulfur; keratolytic agents such as salicylic acid; and mixtures thereof. In an embodiment, the anti-dandruff particulate is a pyridinethione salt.

Pyridinethione particulates are suitable particulate anti-dandruff actives. In an embodiment, the anti-dandruff active is a 1-hydroxy-2-pyridinethione salt and is in particulate form. In an embodiment, the concentration of pyridinethione anti-dandruff particulate ranges from 0.01 wt% to 5 wt%, or from 0.1 wt% to 3 wt%, or from 0.1 wt% to 2 wt%. In an embodiment, the pyridinethione salts are those formed from heavy metals such as zinc, tin, cadmium, magnesium, aluminium and zirconium, generally zinc, typically the zinc salt of 1-hydroxy-2-pyridinethione (known as "zinc pyridinethione" or "ZPT"), commonly 1-hydroxy-2-pyridinethione salts in platelet particle form. In an embodiment, the 1-hydroxy-2-pyridinethione salts in platelet particle form have an average particle size of up to 20 microns, or up to 5 microns, or up to 2.5 microns. Salts formed from other cations, such as sodium, may also be suitable. Pyridinethione anti-dandruff actives are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982.

In an embodiment, in addition to the anti-dandruff active selected from polyvalent metal salts of pyrithione, the composition further comprises one or more anti-fungal and/or anti-microbial actives. In an embodiment, the anti-microbial active is selected from the group consisting of: coal tar, sulfur, fcharcoal, whitfield's ointment, castellani's paint, aluminum chloride, gentian violet, octopirox (piroctone olamine), ciclopirox olamine, undecylenic acid and its metal salts, potassium permanganate, selenium sulphide, sodium thiosulfate, propylene glycol, oil of bitter orange, urea preparations, griseofulvin, 8-hydroxyquinoline ciloquinol, thiobendazole, thiocarbamates, haloprogin, polyenes, hydroxypyridone, morpholine, benzylamine, allylamines (such as terbinafine), tea tree oil, clove leaf oil, coriander, palmarosa, berberine, thyme red, cinnamon oil, cinnamic aldehyde, citronellic acid, hinokitol, ichthyol pale, Sensiva SC-50, Elestab HP-100, azelaic acid, lyticase, iodopropynyl butylcarbamate (IPBC), isothiazalinones such as octyl isothiazalinone, and azoles, and mixtures thereof. In an embodiment, the anti-microbial is selected from the group consisting of: itraconazole, ketoconazole, selenium sulphide, coal tar, and mixtures thereof.

In an embodiment, the azole anti-microbials is an imidazole selected from the group consisting of: benzimidazole, benzothiazole, bifonazole, butaconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and mixtures thereof, or the azole anti-microbials is a triazole selected from the group consisting of: terconazole, itraconazole, and mixtures thereof. When present in the hair care composition, the azole anti-microbial active can be included in an amount of from 0.01 wt% to 5 wt%, or from 0.1 wt% to 3 wt%, or from 0.3 wt% to 2 wt%. In an embodiment, the azole anti-microbial active is ketoconazole. In an embodiment, the sole anti-microbial active is ketoconazole.

Embodiments of the hair care composition may also comprise a combination of anti-microbial actives. In an embodiment, the combination of anti-microbial active is selected from the group of combinations consisting of: octopirox and zinc pyrithione, pine tar and sulfur, salicylic acid and zinc pyrithione, salicylic acid and elubiol, zinc pyrithione and elubiol, zinc pyrithione and climbasole, octopirox and climbasole, salicylic acid and octopirox, and mixtures thereof.

In an embodiment, the composition comprises an effective amount of a zinc-containing layered material. In an embodiment, the composition comprises from 0.001 wt% to 10 wt%, or from 0.01 wt% to 7 wt%, or from 0.1 wt% to 5 wt% of a zinc-containing layered material, by total weight of the composition.

Zinc-containing layered materials may be those with crystal growth primarily occurring in two dimensions. It is conventional to describe layer structures as not only those in which all the atoms are incorporated in well-defined layers, but also those in which there are ions or molecules between the layers, called gallery ions (A.F. Wells "Structural Inorganic Chemistry" Clarendon Press, 1975). Zinc-containing layered materials (ZLMs) may have zinc incorporated in the layers and/or be components of the gallery ions. The following classes of ZLMs represent relatively common examples of the general category and are not intended to be limiting as to the broader scope of materials which fit this definition.

Many ZLMs occur naturally as minerals. In an embodiment, the ZLM is selected from the group consisting of: hydrozincite (zinc carbonate hydroxide), aurichalcite (zinc copper carbonate hydroxide), rosasite (copper zinc carbonate hydroxide), and mixtures thereof. Related minerals that are zinc-containing may also be included in the composition. Natural ZLMs can also occur wherein anionic layer species such as clay-type minerals (e.g., phyllosilicates) contain ion-exchanged zinc gallery ions. All of these natural materials can also be obtained synthetically or formed *in situ* in a composition or during a production process.

Another common class of ZLMs, which are often, but not always, synthetic, is layered double hydroxides. In an embodiment, the ZLM is a layered double hydroxide conforming to the formula $[M^{2+}{}_{1-x}M^{3+}{}_{x}(OH)_2]^{x+}A^{m-}{}_{x/m}\cdot nH_2O$ wherein some or all of the divalent ions ($M^{2+}$) are zinc ions (Crepaldi, EL, Pava, PC, Tronto, J, Valim, JB J. Colloid Interfac. Sci. 2002, 248, 429-42).

Yet another class of ZLMs can be prepared called hydroxy double salts (Morioka, H., Tagaya, H., Karasu, M, Kadokawa, J, Chiba, K Inorg. Chem. 1999, 38, 4211-6). In an embodiment, the ZLM is a hydroxy double salt conforming to the formula $[M^{2+}{}_{1-x}M^{2+}{}_{1+x}(OH)_{3(1-y)}]^{+}\ A^{n-}{}_{(1=3y)/n}\cdot nH_2O$ where the two metal ions ($M^{2+}$) may be the same or different. If they are the same and represented by zinc, the formula simplifies to $[Zn_{1+x}(OH)_2]^{2x+}\ 2x\ A^{-}\cdot nH_2O$. This latter formula represents (where x=0.4) materials such as zinc hydroxychloride and zinc hydroxynitrate. In an embodiment, the ZLM is zinc hydroxychloride and/or zinc hydroxynitrate. These are related to hydrozincite as well wherein a divalent anion replaces the monovalent anion. These materials can also be formed *in situ* in a composition or in or during a production process.

In embodiments having a zinc-containing layered material and a pyrithione or polyvalent metal salt of pyrithione, the ratio of zinc-containing layered material to pyrithione or a polyvalent metal salt of pyrithione is from 5:100 to 10:1, or from 2:10 to 5:1, or from 1:2 to 3:1.

The on-scalp deposition of the anti-dandruff active is at least 1 microgram/cm$^2$. The on-scalp deposition of the anti-dandruff active is important in view of ensuring that the anti-dandruff active reaches the scalp where it is able to perform its function. In an embodiment, the deposition of the anti-dandruff active on the scalp is at least 1.5 microgram/cm$^2$, or at least 2.5 microgram/cm$^2$, or at least 3 microgram/cm$^2$, or at least 4 microgram/cm$^2$, or at least 6 microgram/cm$^2$, or at least 7 microgram/cm$^2$, or at least 8 microgram/cm$^2$, or at least 8 microgram/cm$^2$, or at least 10 microgram/cm$^2$. The on-scalp deposition of the anti-dandruff active is measured by having the hair of individuals washed with a composition comprising an anti-dandruff active, for example a composition pursuant to the present invention, by trained a cosmetician according to a conventional washing protocol. The hair is then parted on an area of the scalp to allow an openended glass cylinder to be held on the surface while an aliquot of an extraction solution is added and agitated prior to recovery and analytical determination of anti-dandruff active content by conventional methodology, such as HPLC.

PRODUCT STABILITY

The addition of histidine to the hair care composition can result in an increase of the composition's pH. It has also been found that the hair care composition containing histidine can turn yellow under longer term extended storage, particularly if the storage is at higher temperatures. A contemplated mechanism for the yellowing of the hair care composition is the formation of Schiff bases with the condensation of an amine with carbonyl compounds found in the hair care composition, for example in perfumes. This reaction can be kinetically driven by pH whereby the reduction in the rate of Schiff base formation can be achieved by lowering the pH to reduce the reactivity of the amine group of the histidine. Citric acid can be included in the composition from 0.01% to 0.5% to lower the pH of the composition. In one embodiment the risk of yellowing of the composition is minimized by reducing the pH of the composition from 7.3 to 4.7.

The term "yellow color" refers to one of the primary colors in the visible spectrum. For example, a yellow colored substance can absorb light in the range of approximately 420-430 nm. Yellow color can be evaluated subjectively, e.g., visually, or objectively, e.g., using a spectrophotometer or a colorimeter. A number of standards and formulas have been developed to evaluate color both subjectively and objectively and can be used to measure yellow color. An example of color scales that can be used to measure yellow color include, but are not limited to, the CIE (International Commission on Illumination) L*a*b* color scale, the CIE L*c*h* color scale, and the Hunter L, a, b color scale. These color scales are based on the Opponent-Colors Theory, which assumes that receptors in the human eye perceive color as a pair of

opposites: light-dark (L* value), red-green (a* value), and yellow-blue (b* value) (see "Hunter L, a, b Versus CIE 1976 L*a*b*," Application Notes, Insight on Color Vol. 13, No. 2 (2008)). Thus, as used herein, the term "b* value" refers to the yellowness or blueness of the composition. A positive b* (+b*) value refers to the yellowness of the composition, whereas a negative b* (-b*) value refers to the blueness of the composition. Additional information regarding color scales and color measurement can be obtained from, e.g., the CIE (www.cie.co.at) or HunterLab (www.hunterlab.com) (Hunter Associates Laboratory, Inc., Reston, VA, USA).

The reduction or decrease in the amount of yellow color in a composition described herein can be measured as a percent decrease in b* value. The measurement of the change in b* values can be performed using a standard colorimeter or spectrophotometer.

TEST METHODS

It is understood that the test methods that are disclosed in the Test Methods Section of the present application should be used to determine the respective values of the parameters of Applicants' invention as such invention is described and claimed herein.

**A. Viscosity Measurements**

A Brookfield viscometer is equipped with a CPE-41 cone and cup assembly. Water bath of cup are set to 30 °C. Gap is set according to the manufacturer's instruction manual. 2.0mL of sample is placed in the center of the cup and a shear rate of 0.3 RPM is set. Viscosity measurement was taken after 210 seconds.

**B. Lab Measurement**

Hair switches can be measured using a Minolta Spectrophotometer CM-3700d. The L*a*b* color space (also referred as CIELab) for measuring colors and determining color differences. The system was defined to provide a uniform color space where equal changes in a color axis corresponds to an equally perceived color difference. In the CIELab space, L* represents lightness, and a* and b* are the chromaticity co-ordinates. The a* and b* axis are defined using a concept of opponent colors, where +a* is the red direction, -a* is the green direction, +b* is the yellow direction, and -b* is the blue direction. a* and b* values are equal to 0 in the center which is achromatic, and increase away from the center. C*, the chroma coordinate, is the perpendicular distance from the lightness axis (more distance being more chroma or movement away from gray tones), h* is the hue angle expressed in degrees, with 0° being a location of the +a* (red) axis, then continuing to 90° for the +b* axis, 180° for - a*, 2700 for -b*, and back to 3600=0°.

Movement along the h* plate denotes color change. When measuring the color reading of a hair switch, the medium area view (MAV) aperture (8 mm diameter) is attached to the CM-3700d instrument aperture. The instrument is calibrated daily to black and white standards. Each switch is measured four times on each side, for a total of 8 eight readings per single switch reading. Ten switch readings are conducted per switch. The first measurement on either side is taken at the top of the switch, with each subsequent reading being one-quarter farther down the mid-line of the switch. The last reading is taken just above the tips of the hair. This method is described in US 2008/0189876 A1.

EXAMPLES

The following examples illustrate the present invention. The exemplified compositions can be prepared by conventional formulation and mixing techniques. It will be appreciated that other modifications of the present invention within the skill of those in the hair care formulation art can be undertaken without departing from the spirit and scope of this invention. All parts, percentages, and ratios herein are by weight unless otherwise specified. Some components may come from suppliers as dilute solutions. The amount stated reflects the weight percent of the active material, unless otherwise specified.

The following are non-limiting examples of hair care compositions encompassed by embodiments of the present invention.

| Components | Ex.1 | Ex.2 | Ex.3 | Ex. 4 | Ex. 5 |
|---|---|---|---|---|---|
| Behenyl trimethyl ammonium chloride | - | - | 2.5 | - | - |
| Behenyl trimethyl ammonium methosulfate | 2.3 | 2.6 | - | 2.6 | 2.3 |
| Dicetyl dimethyl ammonium chloride | - | - | - | - | - |
| Steramidopropyl Dimethylamine | - | - | - | - | - |
| Cetyl alcohol | 1.5 | 1.0 | 1.0 | 1.0 | 1.5 |
| Stearyl alcohol | 3.7 | 2.4 | 2.3 | 2.4 | 3.7 |
| Aminosilicone * | 0.5 | 0.5 | 0.5 | 0.5 | 0.5 |
| Histidine | 0.1 | 0.05 | 0.2 | 0.1 | 0.05 |
| N,N'Ethylene Diamine Disuccinic Acid | | | | | |
| Preservatives | 0.56 | 0.56 | 0.9 | 0.9 | 0.56 |
| Perfume | 0.5 | 0.5 | 0.5 | 0.5 | 0.5 |
| Panthenol | - | - | 0.05 | - | - |
| Panthenyl ethyl ether | - | - | 0.03 | - | - |
| Deionized Water | q.s. to 100% | | | | |
| Method of preparation | I | | | | |

| Components | Ex. 6 | Ex. 7 |
|---|---|---|
| Behenyl trimethyl ammonium methosulfate | 2.0 | 3.0 |
| Steramidopropyl Dimethylamine | - | - |
| Cetyl alcohol | 1.4 | 1.2 |
| Stearyl alcohol | 3.4 | 3.0 |
| Aminosilicone * | 2.0 | 3.0 |
| Histidine | 0.1 | 0.1 |
| Disodium EDTA | | 0.13 |
| N,N'Ethylene Diamine Disuccinic Acid | - | - |
| Preservatives | 0.9 | 0.5 |
| Perfume | 0.5 | 0.5 |
| Deionized Water | q.s. to 100% | q.s. to 100% |

**Comparative Examples**

| Components | Comp. Ex. 1 | Comp. Ex. 2 | Comp. Ex. 3 |
|---|---|---|---|
| Behenyl trimethyl ammonium chloride | - | - | - |
| Behenyl trimethyl ammonium methosulfate | 1.43 | 3.1 | - |
| Dicetyl dimethyl ammonium chloride | 0.50 | - | - |
| Steramidopropyl Dimethylamine | - | - | 2.6 |
| Cetyl alcohol | 0.93 | 1.0 | 1.8 |
| Stearyl alcohol | 2.3 | 2.5 | 3.0 |
| Aminosilicone * | 0.75 | - | - |
| Histidine | - | - | - |
| N,N'Ethylene Diamine Disuccinic Acid | 0.2 | 0.2 | 0.2 |
| Preservatives | 0.56 | 0.56 | - |
| Perfume | 0.5 | - | - |
| Panthenol | - | - | - |
| Panthenyl ethyl ether | - | - | - |
| Deionized Water | q.s. to 100% | | |
| Method of preparation | I | II | I |

Definitions of Components

**[0106]** * Aminosilicone: Terminal aminosilicone which is available from GE having a viscosity of 10,000mPa•s, and having following formula:

$$(R_1)_aG_{3-a}\text{-Si-(-OSiG}_2)_n\text{-O-SiG}_{3-a}(R_1)_a$$

wherein G is methyl; a is an integer of 1; n is a number from 400 to 600; $R_1$ is a monovalent radical conforming to the general formula $C_qH_{2q}L$, wherein q is an integer of 3 and L is - $NH_2$.

Method of Preparation

The conditioning compositions of "Ex. 1" through "Ex. 7" or Comparative Ex. "1" through "3" as shown above can be prepared by any conventional method well known in the art. They are suitably made by one of the following Methods I or II as shown below.

Method I

Cationic surfactants and high melting point fatty compounds are added to water with agitation, and heated to 80°C. The mixture is cooled down to 55°C and gel matrix is formed. Silicones, perfumes and preservatives are added to the gel matrix with agitation. Then, if included polymers are added with agitation at 30°C. Then, if included, other components are added with agitation.

Method II

Cationic surfactants and high melting point fatty compounds are mixed and heated to from 66°C to 85°C to form an oil phase. Separately, water is heated to from 20°C to 48°C to form an aqueous phase. In Becomix® direct injection rotor-stator homogenizer, the oil phase is injected and it takes 0.2 second or less for the oils phase to reach to a high shear field having an energy density of from $1.0x10^5$ J/m$^3$ to $1.0x10^7$ J/m$^3$ where the aqueous phase is already

present. A gel matrix is formed at a temperature of above 50°C to 60°C. Silicones and preservatives are added to the gel matrix with agitation. Then, if included, polymers are added with agitation at 32°C. Then, if included, other components such as perfumes are added with agitation. Then the composition is cooled down to room temperature.

The hair care compositions are generally prepared by conventional methods such as those known in the art of making the compositions. Such methods typically involve mixing of the ingredients in one or more steps to a relatively uniform state, with or without heating, cooling, application of vacuum, and the like. The compositions are prepared such as to optimize stability (physical stability, chemical stability, photostability) and/or delivery of the active materials. The hair care composition may be in a single phase or a single product, or the hair care composition may be in a separate phases or separate products. If two products are used, the products may be used together, at the same time or sequentially. Sequential use may occur in a short period of time, such as immediately after the use of one product, or it may occur over a period of hours or days.

**Stability/Viscosity Data**

To demonstrate the stability of histidine in a hair conditioning composition, the histidine level in Example 1 was varied from 0% up to 0.25% and the viscosity was measured. The viscosity remains stable with -histidine at a level of 0.10% and 0.25%, as demonstrated by the viscosity measurements included in Table 1.

Table 1

| % Histidine Added | Ex. 1 Viscosity in cP at ~30C |
|---|---|
| 0.00 | 5772 |
| 0.10 | 5444 |
| 0.25 | 5608 |

**Claims**

1. A hair care composition comprising:

    a. from 0.025% to 0.25% by weight of the composition of histidine;
    b. a gel matrix comprising:

        i. from 0.1% to 20% of one or more high melting point fatty compounds, by weight of said hair care composition, wherein the one or more high melting point fatty compounds have a melting point of 25°C or higher, and comprise fatty alcohols, wherein the total amount of the one or more high melting point fatty compound ranges from 0.1% to 20%;
        ii. from 0.1% to 10% a cationic surfactant system comprising_cationic surfactant having single straight alkyl chain with 22 carbon atoms, by weight of said hair care composition; and
        iii. at least 20% of an aqueous carrier, by weight of said hair care composition.

2. The hair care composition of claim 1, wherein histidine is present in either D- and L-form or a mixture of the D- and L-forms.

3. The hair care composition of claim 1, wherein said hair care composition comprises from 0.05% to 0.25% of said histidine, by weight of said hair care composition.

4. The hair care composition of claim 1, wherein said hair care composition comprises from 0.08% to 0.15% of said histidine, by weight of said hair care composition.

5. The hair care composition of claim 1, wherein said hair care composition comprises from 0.10% to 0.15% of said histidine, by weight of said hair care composition.

6. The hair care composition of any of the preceding claims, wherein said hair care composition further comprises one or more additional conditioning agents.

7. The hair care composition of claim 6, wherein said one or more additional conditioning agents is a silicone.

8. The hair care composition of any of the preceding claims, wherein said hair care composition further comprises one or more additional benefit agents.

9. The hair care composition of claim 8, wherein said one or more additional benefit agents is selected from the group consisting of anti-dandruff agents, vitamins, chelants, perfumes, brighteners, enzymes, sensates, attractants, anti-bacterial agents, dyes, pigments, bleaches, and mixtures thereof.

10. The hair care composition of any of the preceding claims, further comprising from 0.01% to 0.5% by weight of citric acid.

11. A method for conditioning hair comprising the step of applying an effective amount of the hair care composition as defined in any of the preceding claims to the hair.

**Patentansprüche**

1. Haarpflegezusammensetzung, umfassend:

   a. zu 0,025 Gew.-% bis 0,25 Gew.-% der Zusammensetzung Duftstoff;
   b. eine Gelmatrix, umfassend:

      i. zu 0,1 Gew.-% bis 20 Gew.-% der Haarpflegezusammensetzung eine oder mehrere Fettverbindungen mit hohem Schmelzpunkt, wobei die eine oder die mehreren Fettverbindungen mit hohem Schmelzpunkt einen Schmelzpunkt von 25 °C oder höher aufweisen und Fettalkohole umfassen, wobei der Gesamtgehalt an der einen oder an den mehreren Fettverbindungen mit hohem Schmelzpunkt im Bereich von 0,1 % bis 20 % liegt;
      ii. zu 0,1 Gew.-% bis 10 Gew.-% der Haarpflegezusammensetzung ein kationenaktives Tensidsystem, umfassend ein kationenaktives Tensid, das eine einzelne gerade Alkylkette mit 22 Kohlenstoffatomen aufweist; und
      iii. zu mindestens 20 Gew.-% der Haarpflegezusammensetzung einen wässrigen Träger.

2. Haarpflegezusammensetzung nach Anspruch 1, wobei Histidin entweder in D- und L-Form oder in einer Mischung der D- und L-Formen vorliegt.

3. Haarpflegezusammensetzung nach Anspruch 1, wobei die Haarpflegezusammensetzung zu 0,05 Gew.-% bis 0,25 Gew.-% der Haarpflegezusammensetzung das Histidin umfasst.

4. Haarpflegezusammensetzung nach Anspruch 1, wobei die Haarpflegezusammensetzung zu 0,08 Gew.-% bis 0,15 Gew.-% der Haarpflegezusammensetzung das Histidin umfasst.

5. Haarpflegezusammensetzung nach Anspruch 1, wobei die Haarpflegezusammensetzung zu 0,10 Gew.-% bis 0,15 Gew.-% der Haarpflegezusammensetzung das Histidin umfasst.

6. Haarpflegezusammensetzung nach einem der vorstehenden Ansprüche, wobei die Haarpflegezusammensetzung ferner ein oder mehrere zusätzliche Konditioniermittel umfasst.

7. Haarpflegezusammensetzung nach Anspruch 6, wobei das eine oder die mehreren zusätzlichen Konditioniermittel ein Silikon sind.

8. Haarpflegezusammensetzung nach einem der vorstehenden Ansprüche, wobei die Haarpflegezusammensetzung ferner ein oder mehrere zusätzliche Wirkstoffe umfasst.

9. Haarpflegezusammensetzung nach Anspruch 8, wobei der eine oder die mehreren zusätzlichen Wirkstoffe ausgewählt sind aus der Gruppe bestehend aus Antischuppenmitteln, Vitaminen, Chelants, Duftstoffen, Aufhellern, Enzymen, sinnlich Wahrgenommenem, Lockstoffen, antibakteriellen Mitteln, Farbstoffen, Pigmenten, Bleichmitteln und Mischungen davon.

10. Haarpflegezusammensetzung nach einem der vorstehenden Ansprüche, ferner umfassend zu 0,01 Gew.-% bis 0,5

Gew.-% Zitronensäure.

11. Verfahren zum Konditionieren von Haar, umfassend den Schritt des Auftragens einer wirksamen Menge der Haarpflegezusammensetzung, wie in einem der vorstehenden Ansprüche definiert, auf das Haar.

## Revendications

1. Composition pour soins capillaires, comprenant :

   a. de 0,025 % à 0,25 % en poids de la composition d'histidine ;
   b. une matrice de gel comprenant :

      i. de 0,1 % à 20 % d'un ou plusieurs composés gras à point de fusion élevé, en poids de ladite composition pour soins capillaires, dans laquelle le ou les composés gras à point de fusion élevé ont un point de fusion de 25 °C ou plus élevé, et comprennent des alcools gras, dans laquelle la quantité totale du ou des composés gras à point de fusion élevé va de 0,1 % à 20 % ;
      ii. de 0,1 % à 10 % d'un système tensioactif cationique comprenant un agent tensioactif cationique ayant une unique chaîne alkyle droite avec 22 atomes de carbone, en poids de ladite composition pour soins capillaires ; et
      iii. au moins 20 % d'un véhicule aqueux, en poids de ladite composition pour soins capillaires.

2. Composition pour soins capillaires selon la revendication 1, dans laquelle l'histidine est présente soit dans la forme D et L soit dans un mélange des formes D et L.

3. Composition pour soins capillaires selon la revendication 1, dans laquelle ladite composition pour soins capillaires comprend de 0,05 % à 0,25 % de ladite histidine, en poids de ladite composition pour soins capillaires.

4. Composition pour soins capillaires selon la revendication 1, dans laquelle ladite composition pour soins capillaires comprend de 0,08 % à 0,15 % de ladite histidine, en poids de ladite composition pour soins capillaires.

5. Composition pour soins capillaires selon la revendication 1, dans laquelle ladite composition pour soins capillaires comprend de 0,10 % à 0,15 % de ladite histidine, en poids de ladite composition pour soins capillaires.

6. Composition pour soins capillaires selon l'une quelconque des revendications précédentes, dans laquelle ladite composition pour soins capillaires comprend en outre un ou plusieurs agents de conditionnement supplémentaires.

7. Composition pour soins capillaires selon la revendication 6, dans laquelle ledit ou lesdits agents de conditionnement supplémentaires sont une silicone.

8. Composition pour soins capillaires selon l'une quelconque des revendications précédentes, dans laquelle ladite composition pour soins capillaires comprend en outre un ou plusieurs agents bénéfiques supplémentaires.

9. Composition pour soins capillaires selon la revendication 8, dans laquelle ledit ou lesdits agents bénéfiques supplémentaires sont choisis dans le groupe constitué d'agents antipelliculaires, vitamines, agents chélatants, parfums, azurants, enzymes, sensates, attractifs, agents antibactériens, teintures, pigments, agents de blanchiment et leurs mélanges.

10. Composition pour soins capillaires selon l'une quelconque des revendications précédentes, comprenant en outre de 0,01 % à 0,5 % en poids d'acide citrique.

11. Procédé de conditionnement de cheveux comprenant l'étape consistant à appliquer une quantité efficace de la composition pour soins capillaires telle que définie dans l'une quelconque des revendications précédentes sur les cheveux.

FIGURE 1

| Conditioner Chassis | EDDS level | Observation |
|---|---|---|
| Comparative Ex. 1 | 0.2% | Phase separation |
| Comparative Ex. 2 | 0.2% | 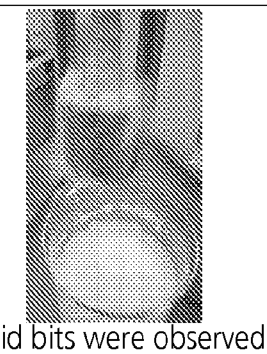 Solid bits were observed upon addition (Phase separation) |
| Comparative Ex. 3 | 0.2% | Viscosity increased by 60Pa at shear stress 950 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4275055 A, Nachtigal
- US 20080292574
- US 20070041929 A
- US 20080292575 A
- US 20070286837 A
- US 6013682 A
- WO 0158986A1 A1
- EP 0874017 A2
- US 4833225 A
- US 20040138400
- US 20040048996
- US 20080292575
- US 5674478 A
- US 5750122 A
- US 4529586 A
- US 4507280 A
- US 4663158 A
- US 4197865 A
- US 4217914 A
- US 4381919 A
- US 4422853 A
- US 4741855 A
- US 2809971 A
- US 3236733 A
- US 3753196 A
- US 3761418 A
- US 4345080 A
- US 4323683 A
- US 4379753 A
- US 4470982 A
- US 20080189876 A1


### Non-patent literature cited in the description


- **TW GRAHAM SOLOMAN.** Organic Chemistry. John Wiley & Son Inc, 1992, 1092-1136
- International Cosmetic Ingredient Dictionary. 1993
- CTFA Cosmetic Ingredient Handbook. 1992
- **A.F. WELLS.** Structural Inorganic Chemistry. Clarendon Press, 1975
- **CREPALDI, EL ; PAVA, PC ; TRONTO, J ; VALIM, JB.** *J. Colloid Interfac. Sci.,* 2002, vol. 248, 429-42
- **MORIOKA, H. ; TAGAYA, H. ; KARASU, M ; KADOKAWA, J ; CHIBA, K.** *Inorg. Chem.,* 1999, vol. 38, 4211-6
- **HUNTER L.** a, b Versus CIE 1976 L∗a∗b∗,'' Application Notes. *Insight on Color,* 2008, vol. 13 (2